(19) Europäisches Patentamt
European Patent Office
Office européen des brevets

(11) Numéro de publication : **0 021 868**
**B1**

# (12) FASCICULE DE BREVET EUROPÉEN

(45) Date de publication du fascicule du brevet : **23.12.81**

(21) Numéro de dépôt : **80400595.7**

(22) Date de dépôt : **30.04.80**

(51) Int. Cl.³ : **C 07 C 41/16**, C 07 C 43/275, C 07 C 43/29, C 07 C 79/35, C 07 C121/75, C 07 C 47/575, C 07 C 69/94, C 07 C 67/31, C 07 C 93/04

## (54) Procédé de préparation d'éthers diaryliques.

(30) Priorité : **18.05.79 FR 7912688**

(43) Date de publication de la demande : **07.01.81 (Bulletin 81/01)**

(45) Mention de la délivrance du brevet : **23.12.81 Bulletin 81/51**

(84) Etats contractants désignés : **AT BE CH DE FR GB IT LI LU NL SE**

(56) Documents cités : **Néant**

(73) Titulaire : **RHONE-POULENC INDUSTRIES**
**Brevets Pharma 25 Quai Paul Doumer**
**F-92408 Courbevoie Cedex (FR)**

(72) Inventeur : **Soula, Gérard**
**33, rue Nungesser**
**F-69330 Meyzieu (FR)**
Inventeur : **Linguenheld, Louis**
**15, Chemin de Puttet**
**F-69230 Saint-Genis Laval (FR)**

(74) Mandataire : **Cazes, Jean-Marie et al**
**RHONE POULENC Service Brevets Chimie et Polymères B.P. 753**
**F-75360 Paris Cedex 08 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

EP 0 021 868 B1

Procédé de préparation d'éthers diaryliques

La présente invention a pour objet un procédé de préparation d'éthers diaryliques ; par réaction d'un halogénobenzène non activé et d'un phénolate ou naphtolate en présence d'un composé du cuivre. Dans la suite de la description et des revendications, on utilisera le terme « phénolate » pour désigner soit un phénolate soit un naphtolate.

Cette réaction est bien connue dans l'art antérieur : c'est la synthèse d'Ullmann des éthers mettant en œuvre, en présence d'un composé du cuivre comme catalyseur, un halogénobenzène non activé (c'est-à-dire ne comportant pas ortho ou para du groupement halogeno un groupement activant sélectivement les positions ortho et para) avec un phénolate.

Plus précisément, on connaît le brevet britannique n° 1 052 390 qui décrit la réaction du m-crésolate de potassium et du bromobenzène en présence de bronze de cuivre activé pour obtenir le phénoxy-3 toluène. Cette réaction a lieu dans le bromobenzène à 220 °C-240 °C. Le principal inconvénient de ce type de procédé est qu'il fait intervenir le dérivé bromé en grande quantité, ce dernier étant utilisé à la fois comme réactif et comme solvant. Or, l'homme de l'art sait bien que le dérivé bromé est onéreux. Un autre inconvénient majeur réside dans la température élevée à laquelle a lieu la réaction.

Il existe cependant des procédés qui permettent l'utilisation du dérivé chloré. On connaît en particulier la demande de brevet japonais 72/104672 qui décrit un procédé de préparation de metaphénoxytoluène selon lequel on fait réagir un sel alcalin de métacrésol avec du chlorobenzène en présence de bases organiques, en utilisant de la poudre de cuivre ou des composés du cuivre comme catalyseur. La réaction a lieu dans la quinoléine à des températures de l'ordre de 200 °C. Le gros inconvénient de ce procédé, quand est envisagée son application à l'échelle industrielle, est sans aucun doute le fait qu'un solvant comme la quinoléine présente de nombreux désavantages qui tiennent à sa difficulté de mise en œuvre et à son prix.

Une autre demande de brevet japonais, la demande 77/035128 décrit un procédé permettant la réaction du chlorobenzène et du m-crésolate en présence du cuivre ou de composés du cuivre sans utiliser de solvant.

La mise en œuvre de ce procédé à l'échelle industrielle soulève de très gros problèmes puisqu'il est nécessaire de travailler sous pression et à des températures élevées (200-250 °C).

On constate donc que dans le cadre d'un procédé de préparation d'éthers diaryliques par réaction d'un halogénobenzène non activé et d'un phénolate en présence d'un composé du cuivre, l'art antérieur ne permet pas une mise en œuvre industrielle aisée et générale de la réaction d'Ullmann. Les besoins non satisfaits par l'art antérieur peuvent s'analyser en trois points pris isolément ou en combinaison. Le premier concerne le solvant : il serait souhaitable de pouvoir utiliser industriellement des solvants ayant une faible toxicité ainsi qu'une bonne stabilité thermique et chimique tout en n'obérant pas l'économie du procédé ; il serait en particulier avantageux dans de nombreux cas de pouvoir utiliser un des réactifs comme solvant. Le second point concerne la température de réaction : il est clair que l'abaissement de la température de réaction serait un avantage très appréciable à la fois au plan technique et au plan économique. Il faut en particulier souligner la nécessité qu'il y a à opérer à des températures les plus basses possibles lorsque les réactifs ou les produits obtenus sont sensibles aux effets thermiques. Le troisième point concerne l'halogénobenzène : comme cela ressort de ce qui précède, il serait souhaitable de remplacer les dérivés bromés par les dérivés chlorés correspondants dans des conditions de réaction semblables.

Les travaux de la demanderesse ont conduit à un procédé de préparation d'éthers diaryliques qui satisfait ces besoins.

La présente invention a donc pour objet un procédé de préparation d'éther diarylique par réaction d'un halogénobenzène non activé et d'un phénolate alcalin en présence d'un composé du cuivre, caractérisé en ce que la réaction a lieu en présence d'au moins un agent séquestrant de formule :

$$N{-}[CHR_1{-}CHR_2{-}O{-}(CHR_3{-}CHR_4{-}O)_n{-}R_5]_3 \quad (I)$$

dans laquelle n est un nombre entier supérieur ou égal à 0 et inférieur ou égal à environ 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-\emptyset$ ou $C_mH_{2m+1}-\emptyset-$, où m est compris entre 1 et 12 ($1 \leq m \leq 12$).

La réaction peut avoir lieu en l'absence ou en présence de solvant.

L'invention repose sur le fait que l'agent sequestrant de formule (I) forme d'une part avec le composé du cuivre et d'autre part avec le phénolate alcalin des complexes qui sont solubles dans le milieu réactionnel alors que le composé du cuivre et le phénolate sont à l'état non complexé insolubles ou très peu solubles dans ledit milieu. Cette complexation a un double effet : en premier lieu, elle permet la solubilisation du catalyseur et du phénolate et de ce fait permet à la réaction d'avoir lieu ; en second lieu, bien que cela ne soit pas complètement expliqué, il semble que la complexation active le système réactionnel de telle sorte que la réaction a lieu dans des conditions beaucoup plus douces que celles de l'art antérieur. C'est ainsi qu'on opère à des températures relativement basses et sous pression

atmosphérique tout en utilisant le dérivé chloré. Il est bien entendu que l'invention s'applique de la même façon aux autres halogénobenzènes comme les bromobenzènes par exemple bien que dans ce cas l'intérêt industriel soit dans la majorité des cas moins marqué.

Lorsqu'on opère sans solvant, le composé du cuivre complexé par l'agent séquestrant et le phénolate alcalin complexé par le même agent séquestrant sont solubles dans l'halogénobenzène, l'agent séquestrant étant soluble dans l'halogénobenzène.

Lorsqu'on opère en présence de solvant, le composé du cuivre complexé par l'agent séquestrant, le phénolate complexé par l'agent séquestrant et l'halogénobenzène sont solubles dans le solvant considéré, l'agent séquestrant étant soluble dans ce même solvant.

Selon un mode de réalisation préférentiel de l'invention, on utilise un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6 et pour lesquels $R_5$ représente un radical alkyle ayant de 1 à 4 atomes de carbone.

On peut citer :

— la tris(oxa-3 butyl)amine de formule :

$$N—(CH_2—CH_2—O—CH_3)$$

— la tris(dioxa-3,6 heptyl)amine de formule :

$$N—(CH_2—CH_2—O—CH_2—CH_2—O—CH_3)_3$$

— la tris(trioxa-3,6,9 décyl)amine de formule :

$$N—(CH_2—CH_2—O—CH_2—CH_2—O—CH_2—CH_2—O—CH_3)_3$$

— la tris(dioxa-3,6 octyl)amine de formule :

$$N—(CH_2—CH_2—O—CH_2—CH_2—O—C_2H_5)_3$$

— la tris(trioxa-3,6,9 undécyl)amine de formule :

$$N—(CH_2—CH_2—O—CH_2—CH_2—O—CH_2—CH_2—O—C_2H_5)_3$$

— la tris(dioxa-3,6 nonyl)amine de formule :

$$N—(CH_2—CH_2—O—CH_2—CH_2—O—C_3H_7)_3$$

— la tris(trioxa-3,6,9 dodécyl)amine de formule :

$$N—(CH_2—CH_2—O—CH_2—CH_2—O—CH_2—CH_2—O—C_3H_7)_3$$

— la tris(dioxa-3,6 décyl)amine de formule :

$$N—(CH_2—CH_2—O—CH_2—CH_2—O—C_4H_9)_3$$

— la tris(trioxa-3,6,9 tridécyl)amine de formule :

$$N—(CH_2—CH_2—O—CH_2—CH_2—O—CH_2—CH_2—O—C_4H_9)_3$$

— la tris(tetra-oxa-3,6,9,12 tridecyl)amine de formule :

$$N—[CH_2—CH_2—O—(CH_2—CH_2—O)_3—CH_3]_3$$

— la tris(hexa-oxa-3,6,9,12,15,18 nonadecyl)amine de formule

$$N—[CH_2—CH_2—O—(CH_2—CH_2—O)—_5CH_3]_3$$

— la tris(dioxa-3,6 méthyl-4 heptyl)amine de formule :

$$N—[CH_2—CH_2—O—(CH—CH_3)—CH_2—O—CH_3]_3$$

— la tris(dioxa-3,6 diméthyl-2,4 heptyl)amine de formule :

$N—[CH_2—(CH—CH_3)—O—(CH—CH_3)—CH_2—O—CH_3]_3$

Les amines utilisées dans le procédé selon l'invention sont connues en tant que telles dans l'art antérieur. C'est ainsi que le brevet français 1 302 365 cite l'obtention des amines tertiaires $N—(CH_2—CH_2—O—CH_3)_3$ et $N—(CH_2—CH_2—O—CH_2—CH_2—O—CH_3)_3$ comme sous produits de la synthèse des amines primaires et secondaires correspondantes, ces amines primaires et secondaires étant des produits intéressants comme intermédiaires en vue de la synthèse de substances pharmaceutiques, comme inhibiteurs de corrosion comme intermédiaires en vue de la synthèse de produits chimiques intéressants en agriculture et comme émulsifiants.

Le procédé selon l'invention est applicable à la réaction d'un halogénobenzène de formule générale :

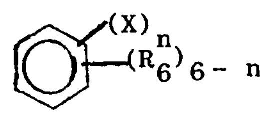 (II)

dans laquelle :

n est supérieur ou égal à 1 et inférieur ou égal à 6 ($1 \leq n \leq 6$).

le ou les radicaux X identiques ou différents sont choisis parmi le groupe comprenant Cl, Br et I.

le ou les radicaux $R_6$ identiques ou différents sont choisis parmi le groupe comprenant :

— l'hydrogène

— les radicaux alkyle et cycloalkyle ayant de 1 à 12 atomes de carbone

— les radicaux alcényle ayant de 3 à 12 atomes de carbone comme les radicaux propényle, nonyle, dodécyle, par exemple

— les radicaux de formules $C_mH_{2m+1}—Ø—$ ; $C_mH_{2m-1}—Ø—$ ; et $Ø—C_mH_{2m}—$ où m est un nombre entier compris entre 1 et 12 ($1 \leq m \leq 12$) et où Ø peut être substitué

— les radicaux alkoxy ayant de 1 à 12 atomes de carbone et les radicaux phénoxy

— les radicaux $—C_mH_{2m}—OH$ et $—C_mH_{2m}$ OR où m est un nombre entier compris entre 1 et 12 ($1 \leq m \leq 12$) et où R est un radical alkyle ayant de 1 à 12 atomes de carbone ou un radical phényle.

— les radicaux alkylthio ayant de 1 à 12 atomes de carbone et les radicaux phénylthio

— les radicaux $C_pH_{2p+1-q}$ Fq, p étant compris entre 1 et 4 ($1 \leq p \leq 4$) et q étant compris entre 3 et 9 ($3 \leq q \leq 9$) comme $—CF_3$ et $—CH_2—CF_3$ par exemple

— les radicaux

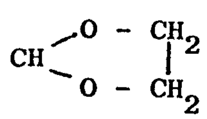 et 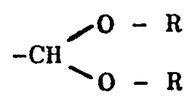 où

R est un radical alkyle ayant de 1 à 12 atomes de carbone ou un radical phényle

— et les radicaux $—NO_2$, $—SO_3M$, —CN, $—CO_2M$, $—CO_2R$, —COR, —COH où M représente un métal alcalin et où R représente un radical alkyle ayant de 1 à 12 atomes de carbone ou un radical phényle.

Lorsque $R_6$ est en position ortho ou para d'un substituant X il ne peut représenter l'un des radicaux $—NO_2$, $—SO_3M$, —CN, $—CO_2M$, $—CO_2R$, —COR et $—SO_2R$ définis ci-dessus, ces radicaux activant en effet sélectivement ces positions ortho et para.

Les phénolates pouvant être mis en œuvre selon le procédé de l'invention ont pour formule :

$$Ar(O\ M')_r \qquad (III)$$

dans laquelle :

Ar représente un radical phényle ou naphtyle éventuellement substitué

M' représente un cation choisi parmi le groupe comprenant les cations dérivés des métaux alcalins et r est un nombre entier compris entre 1 et 3 ($1 \leq r \leq 3$)

Plus particulièrement, l'invention concerne les composés de formules :

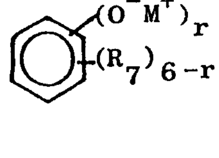 (IIIa) et 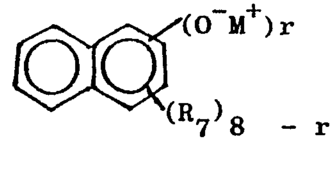 (IIIb)

dans lesquelles :

r est égal à 1 ou 2

le ou les cations $M^+$, identiques ou différents, sont choisis parmi le groupe comprenant $Li^+$, $Na^+$, $K^+$.

le ou les radicaux $R_7$, identiques ou différents, sont choisis parmi le groupe comprenant :

— l'hydrogène

— les radicaux alkyle et cycloalkyle ayant de 1 à 12 atomes de carbone

— les radicaux alcényle ayant de 3 à 12 atomes de carbone comme les radicaux propényle, nonyle, dodécyle, par exemple

— les radicaux de formules $C_mH_{2m+1}Ø—$ ; $C_mH_{2m-1}Ø—$ ; et $Ø—C_mH_{2m}—$ où m est un nombre entier compris entre 1 et 12 ($1 \leq m \leq 12$) et où Ø peut être substitué

— les radicaux alkoxy ayant de 1 à 12 atomes de carbone et les radicaux phénoxy

— les radicaux $—C_mH_{2m}—OH$ et $—C_mH_{2m}\,OR$ où m est un nombre entier compris entre 0 et 12 ($0 \leq m \leq 12$) et où R est un radical alkyle ayant de 1 à 12 atomes de carbone ou un radical phényle

— les radicaux alkylthio ayant de 1 à 12 atomes de carbone et les radicaux phénylthio

— les radicaux $C_pH_{2p+1-q}F_q$, p étant compris entre 1 et 4 ($1 \leq p \leq 4$) et q étant compris entre 3 et 9 ($3 \leq q \leq 9$) comme $—CF_3$ et $—CH_2—CF_3$ par exemple

— les radicaux

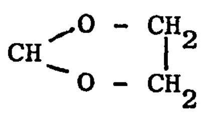 et 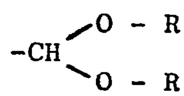

où R est un radical alkyle ayant de 1 à 12 atomes de carbone ou un radical phényle

— les radicaux Cl et F

— et les radicaux $—NO_2$, $NH_2$, NHR, NRR, $—SO_3M$, —CN, $—CO_2M$, $—CO_2R$, —COR, —COH, $—SO_2R$ où M représente un métal alcalin et où R représente un radical alkyle ayant de 1 à 12 atomes de carbone ou un radical phényle.

L'invention vise plus particulièrement, mais non exclusivement, la réaction d'un composé de formule II ne contenant qu'un substituant X avec un composé de formule III ne contenant qu'un substituant $O^-M^+$ ainsi que la réaction d'un composé de formule II contenant un substituant X avec un composé de formule III contenant plusieurs substituants $O^-M^+$ et inversement c'est-à-dire un composé de formule II contenant plusieurs substituants X avec un composé de formule III contenant un substituant $O^-M^+$.

On peut citer comme exemples d'halogénobenzènes de formule II les composés suivants :

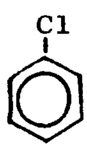 ; 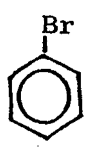 ; 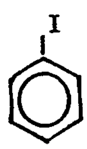 ; 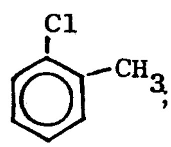 ; 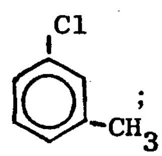 ; 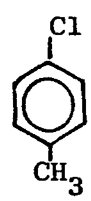 ; 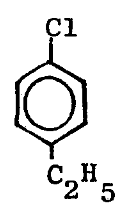

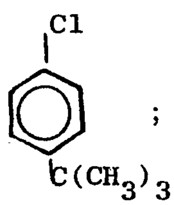 ; 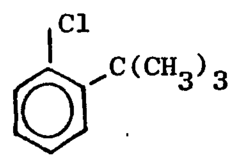 ; 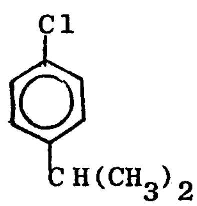 ; 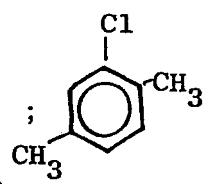 ; 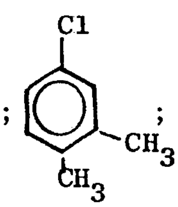 ;

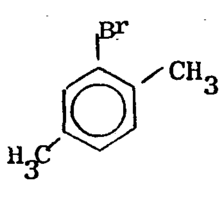 ; 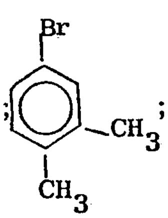 ; 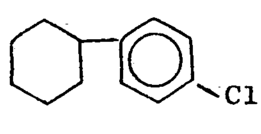 ; 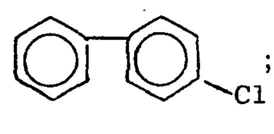 ;

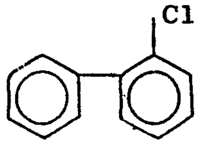 ; 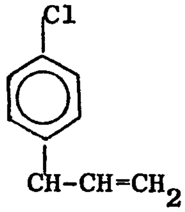 ; 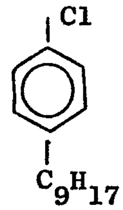 ; 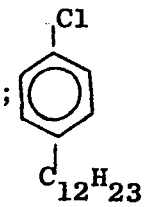 ;

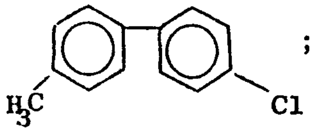 ; 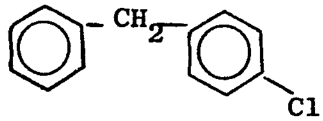 ; 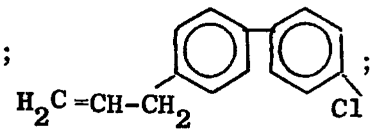 ;

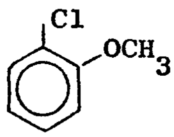 ; 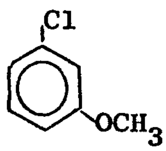 ; 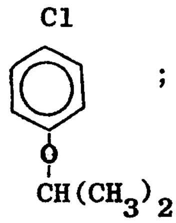 ; 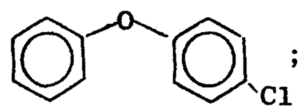 ;

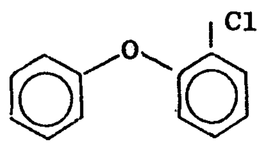 ; 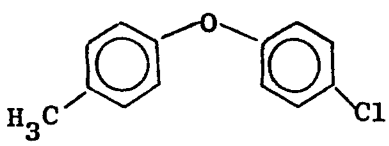 ; 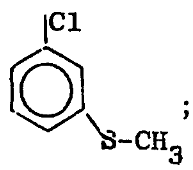 ;

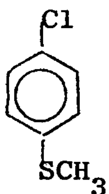 ; 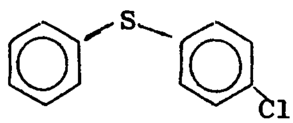 ; 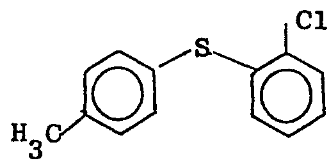 ; 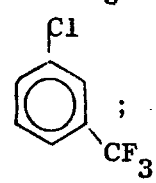 ;

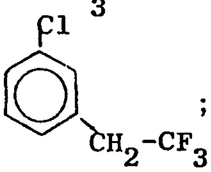 ; 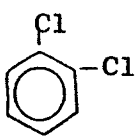 ; 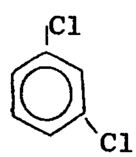 ; 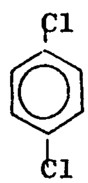 ; 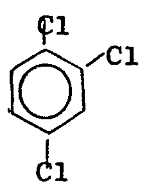 ;

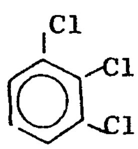 ; 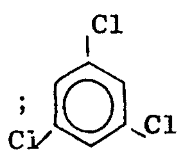 ; 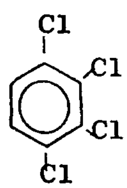 ; 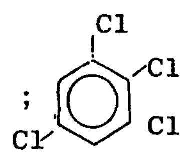 ; 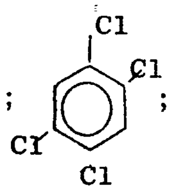 ;

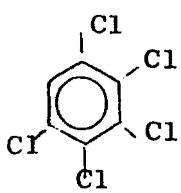 ; 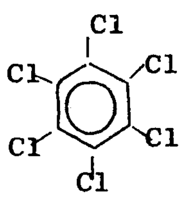 ; 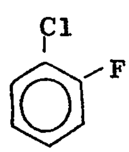 ; 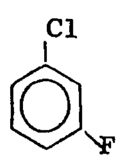 ; 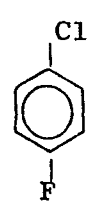 ;

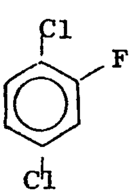 ; 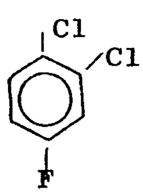 ; 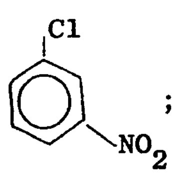 ; 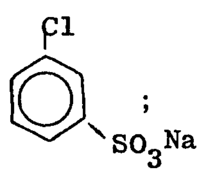 ;

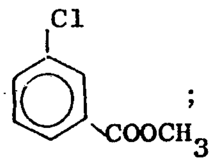 ; 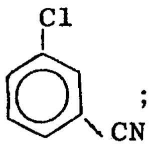 ; 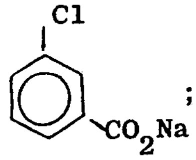 ; 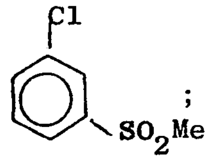 ;

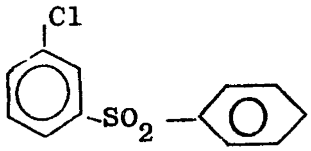 ; 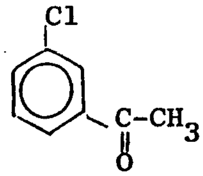 ; 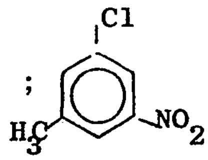 ; 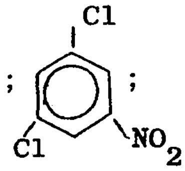 ;

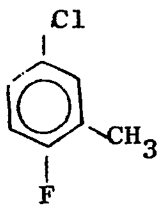 ; 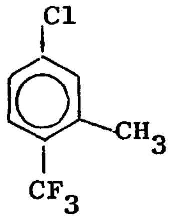 ; 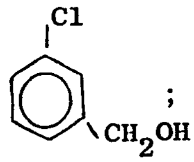 ; 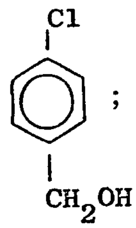 ; 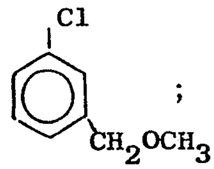 ;

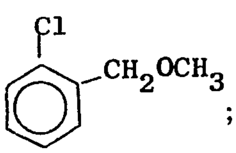 ; 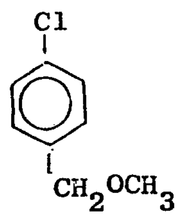 ; 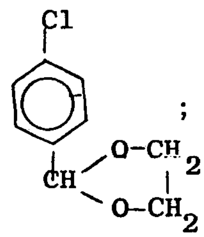 ; 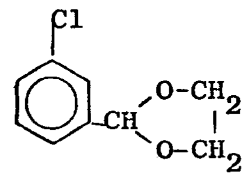 ;

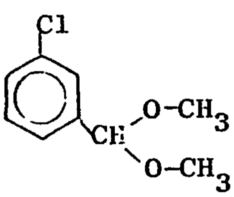 ; 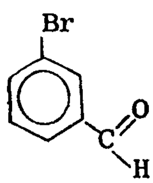 ; 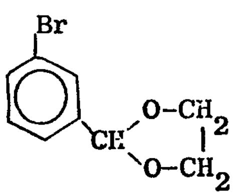 ; 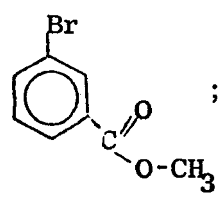 ;

On peut citer comme exemples de phénolates de formules IIIa et IIIb les composés dérivés des phénols et naphtols suivants :

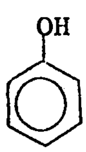 ; 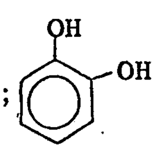 ; 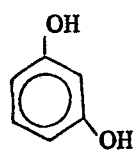 ; 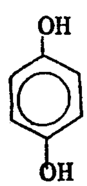 ; 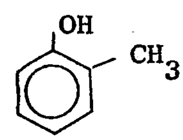 ;

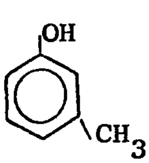 ; 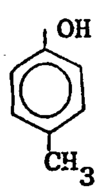 ; 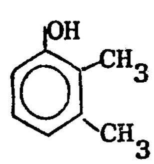 ; 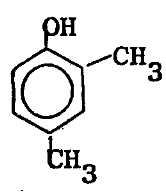 ; 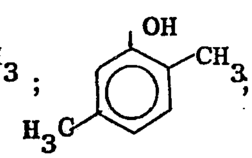 ,

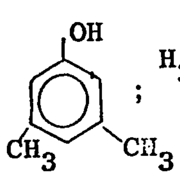 ; 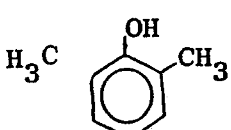 ; 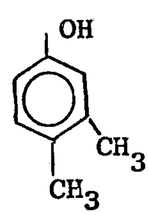 ; 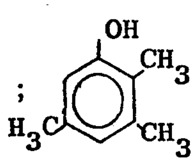 ; 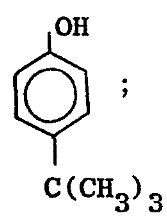 ;

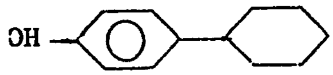 ; 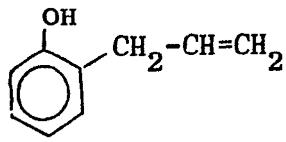 ; 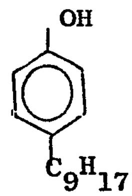 ;

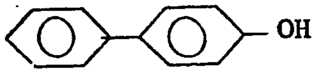 ; 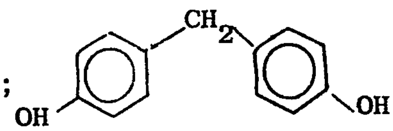 ;

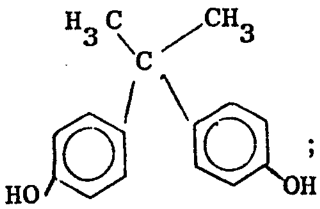 ; 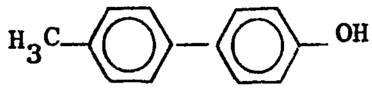 ; 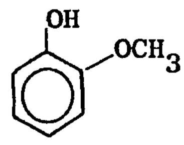 ;

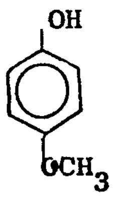 ; 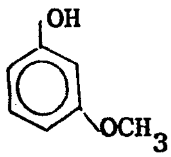 ; 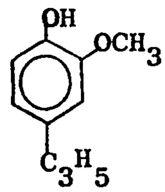 ; 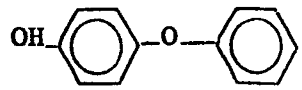 ;

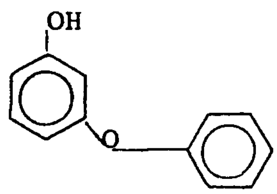 ; 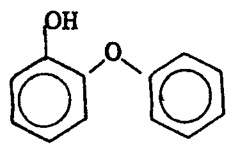 ; 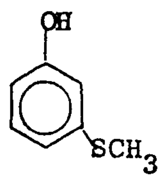 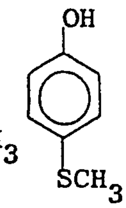

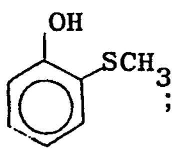 ; 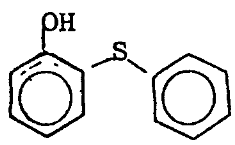 ; 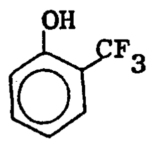 ; 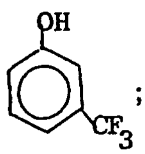 ; 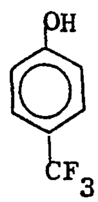

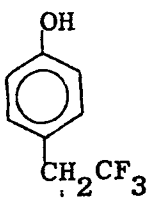 ; 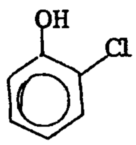 ; 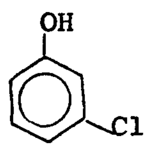 ; 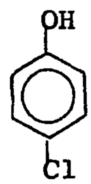 ; 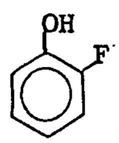 ;

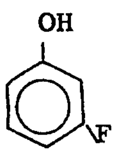 ; 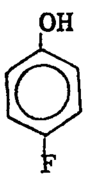 ; 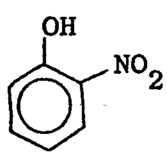 ; 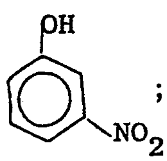 ; 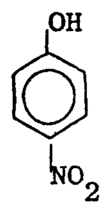 ; 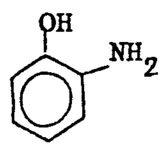

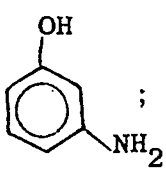 ; 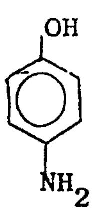 ; 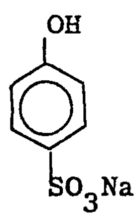 ; 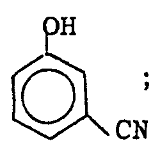 ; 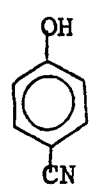 ; 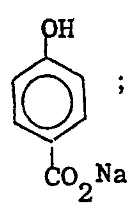 ;

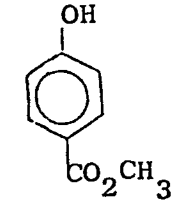 ; 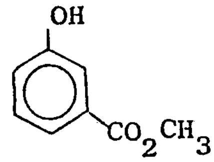 ; 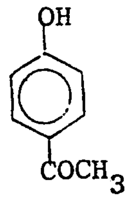 ; 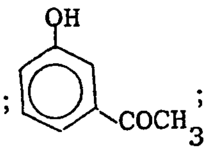 ; 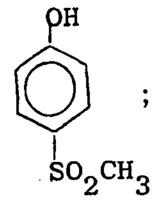 ;

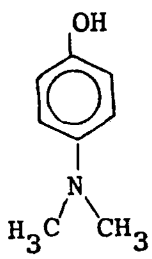 ; 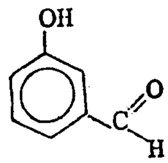 ; 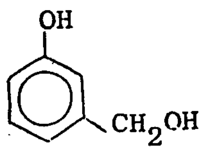 ; 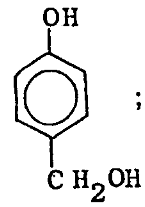 ;

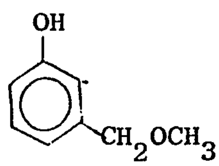 ; 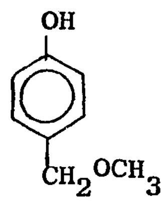 ; 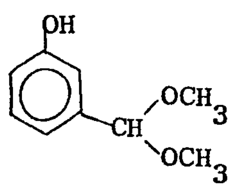 ; 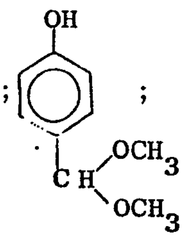 ;

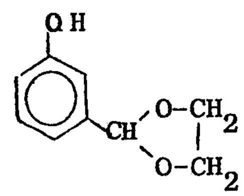 ; 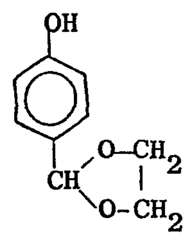 ; 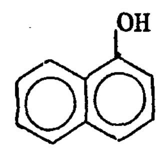 ; 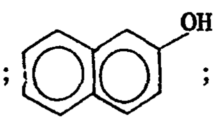 ;

Le choix de l'agent séquestrant le plus adapté à la mise en œuvre du procédé selon l'invention doit être fait en tenant compte de la taille du cation $M^+$. Plus la taille du cation sera importante, plus le nombre d'atomes d'oxygène contenus dans la molécule de l'agent séquestrant devra être élevé. C'est ainsi que si on utilise un phénolate de potassium on préfèrera utiliser la tris(trioxa-3,6,9 décyl)amine alors qu'avec le sel de sodium correspondant la tris(dioxa-3,6 heptyl)amine sera préférée.

Le solvant, lorsqu'on en utilise un, doit répondre à un certain nombre de conditions : il faut d'abord qu'il solubilise l'agent séquestrant (ce dernier est soluble dans la plupart des solvants usuels) ; il faut aussi qu'il soit inerte chimiquement vis-à-vis des sels à dissoudre. Il faut aussi noter que pour obtenir la meilleure mise en œuvre du procédé selon l'invention, plus le solvant choisi aura un caractère apolaire marqué, plus l'agent séquestrant devra avoir un caractère lipophile marqué (c'est-à-dire plus l'agent séquestrant devra contenir d'atomes de carbone).

On peut, par exemple utiliser comme solvant :

l'oxyde de diphényle, l'anisole, le toluène, les polyéthers de glycol, le benzène, les xylènes.

Les composés du cuivre utilisables comme catalyseurs sont connus dans l'art antérieur. On peut citer : Cu Cl, Cu Br, Cu I, Cu O $COCH_3$, $Cu_2O$. Selon un mode de mise en œuvre préféré, on utilise Cu Cl ou Cu Br.

Le procédé selon l'invention est mis en œuvre à une température comprise entre 50 °C et 200 °C environ. D'une façon préférentielle, on opère à une température comprise entre environ 100 et environ 180 °C.

Comme cela a été dit plus haut, on opère généralement à la pression atmosphérique. Bien entendu, les pressions inférieures ou supérieures à la pression atmosphérique ne sont pas exclues par la présente invention.

On utilise l'agent séquestrant en quantité telle que le rapport molaire du composé du cuivre à l'agent séquestrant de formule I est de préférence compris entre environ 0,05 et 10. Encore plus préférentiellement, ce rapport est compris entre environ 0,1 et 5.

Le rapport molaire du composé du cuivre au phénolate est de préférence compris entre environ 0,005 et environ 0,15. Il est encore plus préférentiellement compris entre 0,01 et 0,1 environ.

Le rapport molaire de l'halogénobenzène au phénolate est compris de préférence entre environ 0,8 et

environ 50. Plus préférentiellement, ce rapport est compris entre 0,9 environ et 30 environ. Les hautes valeurs de ce rapport correspondent au cas où l'halogénobenzène sert aussi de solvant.

Les composés obtenus selon la présente invention ont les formules générales IV suivantes :

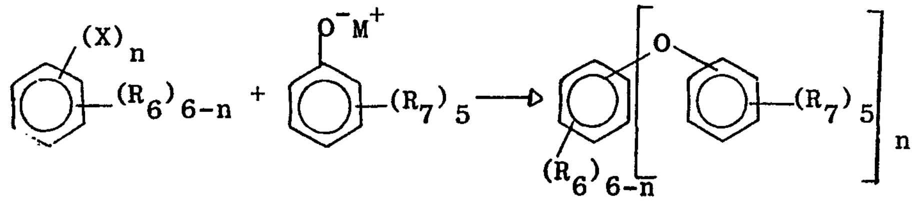
(IVa)

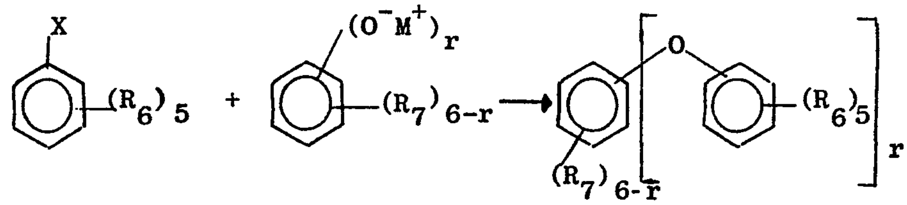
(IVb)

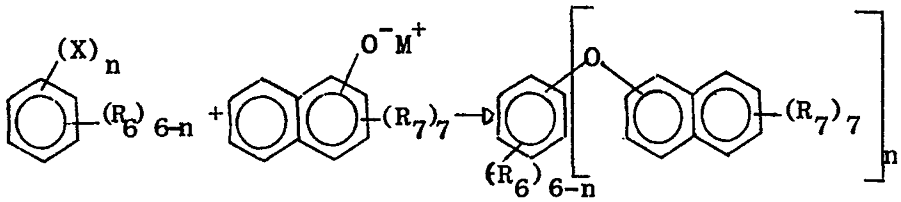
(IVc)

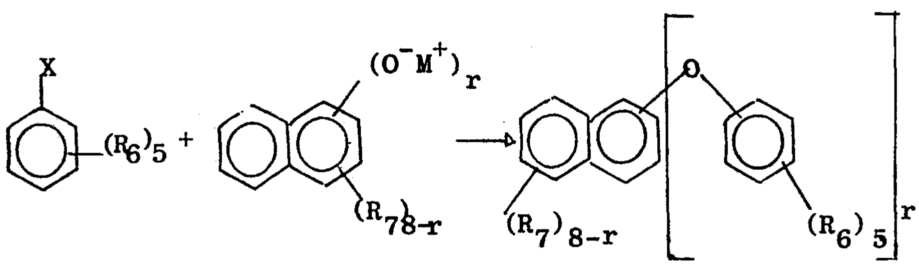
(IVd)

On peut citer comme exemples de composés de formule IV, les composés suivants :

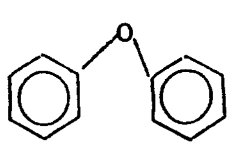
;
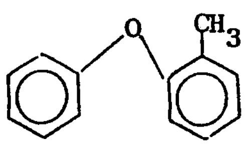
;
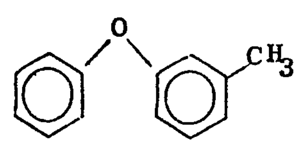
;

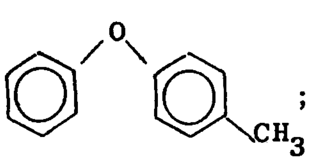
;
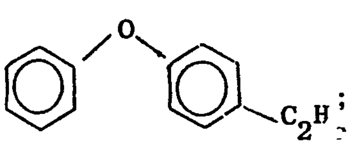
;
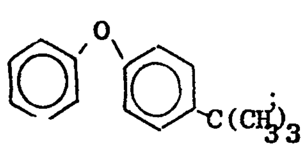
;

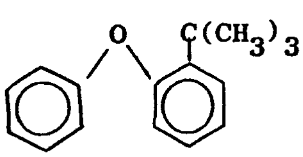
;
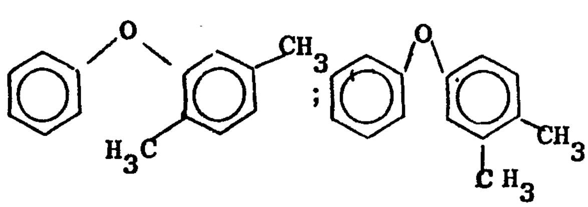
;

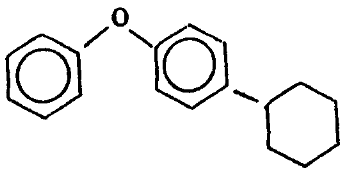 ; 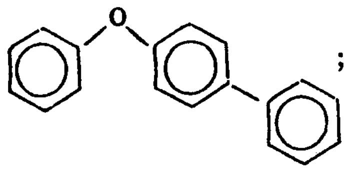 ; 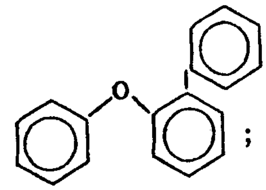 ;

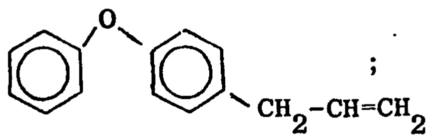 ; 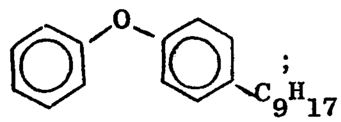 ;

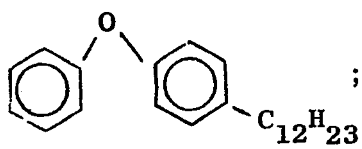 ; 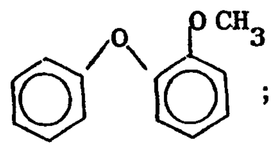 ; 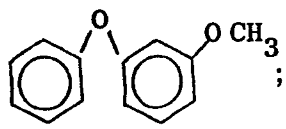 ;

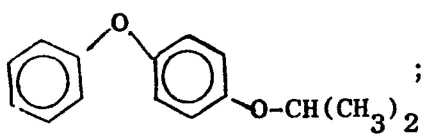 ; 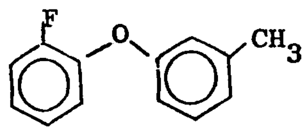 ;

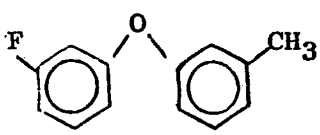 ; 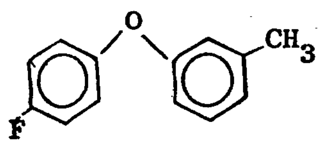 ; 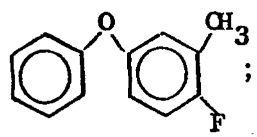 ;

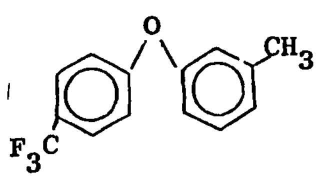 ; 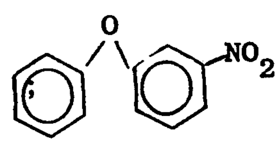 ; 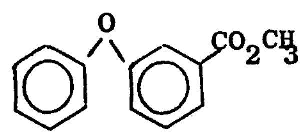 ;

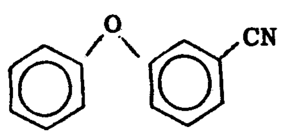 ; 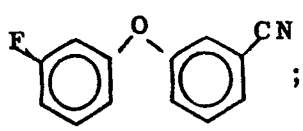 ; 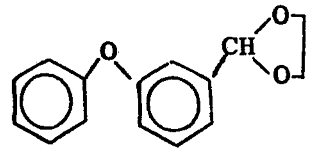 ;

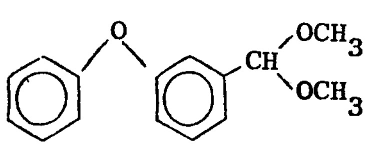 ; 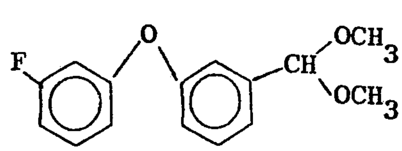 ;

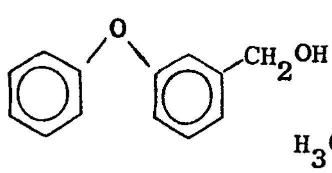 ; 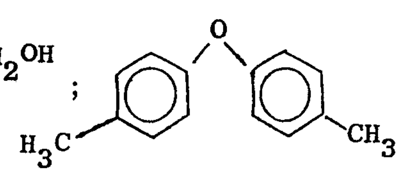 ;

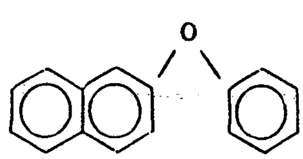 ; 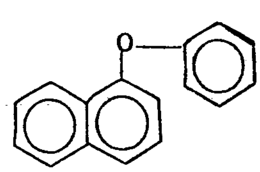 ;

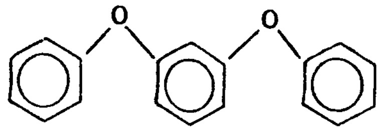 ; 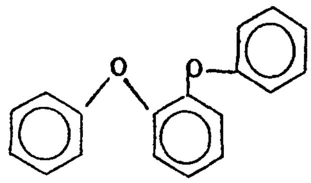 ;

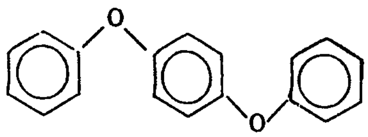 ; 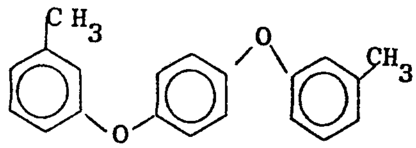 ;

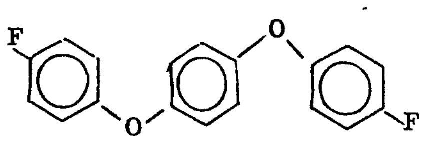 ; 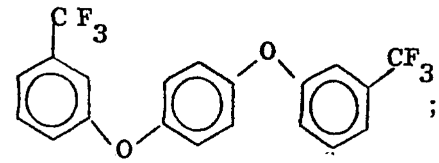 ;

Ils sont utiles notamment comme intermédiaires pour la synthèse de composés ayant une activité phytosanitaire et pharmaceutique.

Les agents séquestrants de formule I utilisés dans le procédé selon l'invention peuvent être préparés par condensation d'un sel de formule :

$$R_5\ (O - \overset{R_4}{\overset{|}{CH}} - \overset{R_3}{\overset{|}{CH}})_n - O{-}M$$

où $R_3$, $R_4$, $R_5$ et n ont la signification précédente et où M représente un atome de métal alcalin choisi parmi le sodium, le potassium et le lithium, soit sur une amine de formule générale :

$$N{-}(\overset{R_1}{\overset{|}{CH}} - \overset{R_2}{\overset{|}{CH}} - X)_3$$

dans laquelle $R_1$ et $R_2$ ont la signification précédente et X représente le chlore ou le brome, soit sur le chlorhydrate ou le bromhydrate correspondant.

Le rapport molaire sel de métal alcalin/amine est compris entre environ 3 et environ 5.

L'opération de condensation est réalisée à une température comprise entre 100 et 150 °C pendant 1 à 15 h en présence d'un solvant qui peut être par exemple le chlorobenzène ou de préférence le monoalkyléther d'éthylène glycol de formule $R_5{-}(O{-}CHR_4{-}CHR_3)_n{-}OH$.

On opère de préférence de telle sorte qu'on ait une solution contenant de 2 à 5 moles de sel de métal alcalin par litre de solvant.

Le mélange en fin de réaction contient principalement l'amine tertiaire de formule :

$$N{-}\left[\overset{R_1}{\overset{|}{CH}}{-}\overset{R_2}{\overset{|}{CH}}{-}O{-}(\overset{R_3}{\overset{|}{CH}}{-}\overset{R_4}{\overset{|}{CH}}{-}O)_n{-}R_5\right]_3$$

mais contient aussi en faible proportion de l'amine secondaire correspondant :

$$HN{-}\left[\underset{R_1}{\underset{|}{CH}}{-}\underset{R_2}{\underset{|}{CH}}{-}O{-}(\underset{R_3}{\underset{|}{CH}}{-}\underset{R_4}{\underset{|}{CH}}{-}O)_n{-}R_5\right]_2$$

et des traces d'amine primaire :

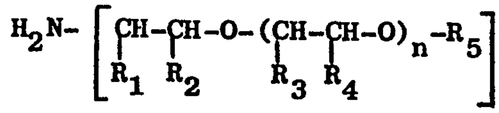

Les amines tertiaires, secondaires et primaires sont généralement respectivement dans le rapport 90 : 8 : 2 après distillation.

On peut utiliser dans le procédé selon l'invention directement le mélange ci-dessus obtenu après première distillation, c'est-à-dire contenant les trois types d'amines.

On préfère pour une meilleure mise en œuvre de l'invention effectuer une distillation plus poussée du mélange ci-dessus afin d'obtenir une amine tertiaire sensiblement pure.

D'autres caractéristiques et avantages de l'invention apparaîtront plus clairement à la lecture des exemples qui vont suivre. Ces exemples ne sauraient en aucune manière être considérés comme une limitation de l'invention.

Exemple 1

Préparation du métaphénoxytoluène

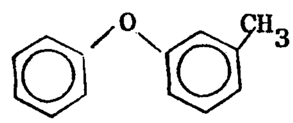

à partir de métacrésolate de sodium

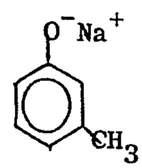

et de chlorobenzène

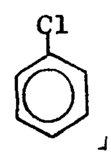

en présence de chlorure cuivreux Cu Cl et

a) en présence de tris(dioxa-3,6 octyl)amine de formule $N(CH_2—CH_2—O—CH_2—CH_2—O—C_2H_5)_3$.

Dans un ballon tricol de 2 l équipé d'une agitation, d'un thermomètre, d'une ampoule de coulée et d'un séparateur de fraction, on charge 216 g (2 moles) de métacrésol, 80 g (2 moles) de soude, 60 g d'eau et 1 250 g (11 moles) de chlorobenzène. On chauffe alors jusqu'à 133 °C tandis que l'eau est entraînée par azéotropie. La bouillie de crésolate de soude est fluide à 135 °C. A ce moment, on met un courant de gaz inerte (azote) et on charge 18 g (0,18 mole) de chlorure cuivreux et 38 g (0,104 mole) de tris(dioxa-3,6 octyl)amine. On maintient au reflux pendant 6 h tandis que l'avancement de la réaction est suivi par chromatographie en vérifiant la disparition du chlorobenzène et l'apparition du phénoxytoluène. Après 6 h à 135 °C, le taux de transformation est de 89 % et le rendement de 97 %.

Après refroidissement, le chlorure de sodium est extrait à l'eau acide, puis à l'eau alcaline et la masse organique est soumise à distillation pour obtenir 302 g de métaphénoxytoluène $PEb_5$ : 120 °C, $d^{20}_4$ : 1,045.

b) en présence de tris(dioxa-3,6 heptyl)amine de formule $N(CH_2—CH_2—O—CH_2—CH_2—O—CH_3)_3$.

Dans le même appareillage que ci-dessus, on charge 216 g (2 moles) de métacrésol, 60 g (1,5 moles) de soude, 28 g (0,5 mole) de potasse, 60 g d'eau et 1 250 g (11 moles) de chlorobene. On opère la salification comme dans l'exemple 1a puis après avoir mis la masse sous un courant d'hydrogène, on ajoute 17 g (0,17 mole) de chlorure cuivreux et 30 g (0,093 mole) de tris(dioxa-3,6 heptyl)amine. On maintient au reflux pendant 6 h tout en suivant l'avancement de la réaction. Après ce laps de temps, le taux de transformation du crésol atteint 90 % et le rendement 97 %.

Essai comparatif

On opère comme ci-dessus mais sans ajouter d'agent séquestrant selon l'invention. Après 6 h, le degré d'avancement de la réaction n'est que 5 % et il faut attendre 106 h de reflux pour obtenir un taux de transformation de 50 %.

Exemple 2

Préparation de métaphénoxytoluène

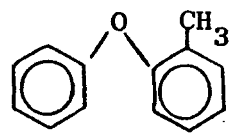

à partir de phénolate de sodium

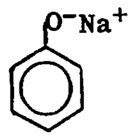

et de chloro-3 toluène

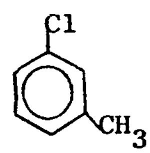

en présence de chlorure cuivreux Cu Cl et de tris(dioxa-3,6 octyl)amine $N{-}(CH_2{-}CH_2{-}O{-}CH_2{-}CH_2{-}O{-}C_2H_5)_3$.

Dans un erlen de 100 cm$^3$ agité par un barreau magnétique sous couverture d'azote, on charge 1,16 g (0,01 mole) de phénate de sodium, 20 g (0,158 mole) de chloro-3 toluène, 0,099 g (0,001 mole) de chlorure cuivreux et 0,36 g (0,001 mole) de tris(dioxa-3,6 octyl)amine. Après 4 h de reflux, le taux de transformation atteint 91 %.

Essai comparatif

On opère comme ci-dessus mais sans ajouter de tris(dioxa-3,6 octyl)amine. Après 4 h de reflux le taux de transformation atteint 0,6 %.

Exemple 3

Préparation de l'orthophénoxy toluène

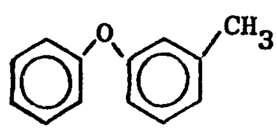

à partir d'orthocrésolate de sodium

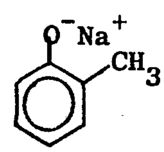

et de chlorobenzène

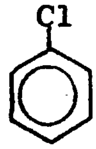

en présence de chlorure cuivreux Cu Cl et de tris(dioxa-3,6 octyl)amine $N(CH_2—CH_2—O—CH_2—CH_2—O—C_2H_5)_3$.

Dans un erlen de 100 $cm^3$ agité par un barreau magnétique, on charge 1,30 g (0,01 mole) d'orthocrésolate de sodium, 20 g (0,18 mole) de chlorobenzène, 0,099 g (0,001 mole) de chlorure cuivreux et 0,36 g (0,001 mole) de tris(dioxa-3,6 octyl)amine. Après 6 h de reflux, le taux de transformation atteint 92 %.

Essai comparatif

Le même essai est réalisé sans addition de tris(dioxa-3,6 octyl)amine. Après 6 h de reflux, le taux de transformation n'est que de 1,2 %.

Exemple 4

Préparation du diphénoxy-1,2 benzène

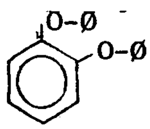

à partir de phénolate de sodium Ø—$O^-Na^+$ et d'orthodichlorobenzène

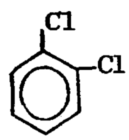

en présence de chlorure cuivreux et de tris(dioxa-3,6 octyl)amine.

Dans un erlen de 100 ml agité par un barreau magnétique on charge 2,32 g (0,002 mole) de phénate de sodium 1,47 g (0,01 mole) d'o-dichlorobenzène, 0,2 g (0,002 mole) de chlorure cuivreux et 0,72 g (0,002 mole) de tris(dioxa-3,6 octyl)amine dans 20 g d'anisole. Après 20 h au reflux, le taux de transformation est de 60 % en diphénoxy-1,2 benzène et de 5 % en chloro-1 phénoxy-2 benzène.

Essai comparatif

Le même essai est réalisé sans ajouter de tris(dioxa-3,6 octyl)amine : le taux de transformation est nul après 20 h au reflux de l'anisole.

Exemple 5

Préparation de méthyl-3' phénoxy-2 fluorobenzène

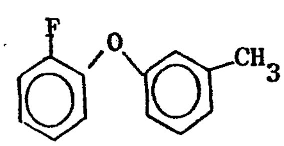

à partir de chloro-1 fluoro-2 benzène

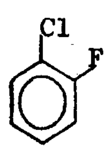

et de métacrésolate de sodium

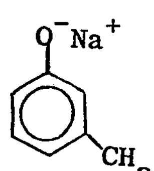

en présence de chlorure cuivreux Cu Cl et de tris(dioxa-3,6 octyl)amine.

Dans un erlen de 100 ml agité par un barreau magnétique et sous courant d'hydrogène, on charge 20 g (0,15 mole) de chloro-1 fluoro-2 benzène, 1,37 g (0,01 mole) de m-crésolate de sodium, 0,099 g (0,001 mole) de chlorure de cuivre et 0,36 g (0,001 mole) de tris(dioxa-3,6 octyl)amine. On chauffe à reflux pendant 24 h. Le taux de transformation en fluoro-1 méthyl-3' phénoxy-2 benzène atteint 75 %.

Essai comparatif

On opère comme ci-dessus mais sans ajouter de tris(dioxa-3,6 octyl)amine. Le taux de transformation est de 11 % après 24 h de reflux.

Exemple 6

Préparation de fluoro-3' phénoxy-3 toluène

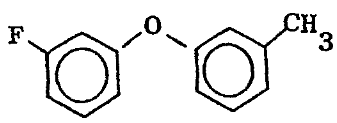

à partir de chloro-3 toluène

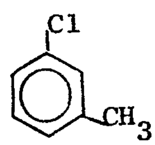

et de métafluorophénolate de sodium

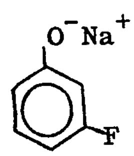

en présence de chlorure cuivreux Cu Cl et de tris(dioxa-3,6 octyl)amine.

Dans un ballon de 500 ml équipé d'une agitation et chauffé par bain d'huile, on charge 110 g (0,87 mole) de chloro-3 toluène, 8 g (0,06 mole) de métafluorophénate de sodium, 0,6 g (0,006 mole) de chlorure cuivreux et 2,2 g (0,006 mole) de tris(dioxa-3,6 octyl)amine. On chauffe 8 h 30 à reflux (180 °C). Le taux de conversion en m-fluoro-3'phénoxytoluène atteint alors 65,9 %.

Essai comparatif

On opère comme ci-dessus mais sans ajouter d'agent séquestrant : le taux de transformation est de 5 %.

Exemple 7

Préparation de métaphénoxybenzonitrile

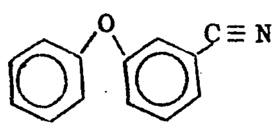

à partir de chloro-3 benzonitrile

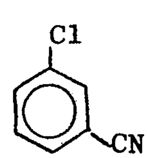

et de phénolate de sodium

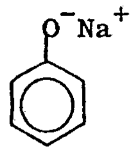

en présence de chlorure cuivreux Cu Cl et de tris(dioxa-3,6 octyl)amine et

a) en présence d'anisole :

Dans un ballon de 1 l, équipé comme dans l'exemple 1, on charge 100 g (0,73 mole) de chloro-3 benzonitrile, 98 g (0,84 mole) de phénate de sodium, 7,2 g (0,072 mole) de chlorure cuivreux, 10,3 g (0,028 mole) de tris(dioxa-3,6 octyl)amine et 570 g d'anisole. On chauffe alors à reflux (155 °C) sous atmosphère d'azote pendant 6 h. Le taux de transformation est de 85 % et le rendement en m-phénoxybenzonitrile distillé est de 70 %.

b) en présence d'oxyde de diphényle :

On opère comme sous a) en remplaçant l'anisole par l'oxyde de phényle qui permet de chauffer à 180 °C pendant 6 h. Le taux de transformation est alors de 95 % et le rendement en m-phénoxybenzonitrile distillé, de 70 %.

## Essai comparatif

On opère comme sous b) sans ajouter de tris(dioxa-3,6 octyl)amine. Le taux de transformation après 6 h est de 15 %.

## Exemple 8

Préparation de métaphénoxybenzonitrile

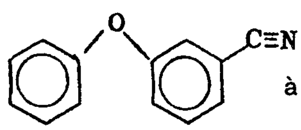 à partir de 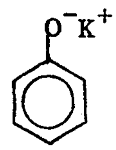 et de 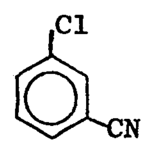

en présence de chlorure cuivreux Cu Cl et de tris(trioxa-3,6,9 décyl)amine.

Dans un ballon de 3 l équipé comme dans l'exemple 1, on charge 192 g (3,43 moles) de potasse, 252 g d'eau, 320 g (3,8 moles) de phénol et 675 g d'anisole. On opère la salification en chauffant à reflux pour éliminer l'eau par azéotropie. On met ensuite l'appareillage sous azote et on charge 460 g (3,34 moles) de chlorobenzonitrile dissous dans 500 g d'anisole, 36 g (0,36 mole) de chlorure cuivreux et 26 g (0,057 mole) de tris(trioxa-3,6,9 décyl)amine. On maintient au reflux (140 °C) pendant 6 h : le taux de conversion atteint 80 %.

Après refroidissement de la masse, on extrait les chlorures par de l'eau acidulée et après décantation de la couche aqueuse, on récupère par distillation l'anisole, le phénol non réagi et le m-phénoxybenzonitrile. $PEb_{0,5}$ : 127 °C — Rendement : 73 %.

## Exemple 9

Préparation du métaphénoxynitrobenzène

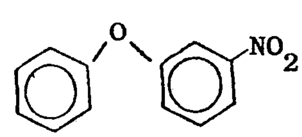

à partir de métachloronitrobenzène

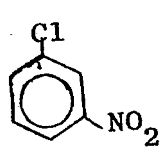

et de phénolate de sodium

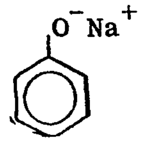

en présence de chlorure cuivreux Cu Cl et de tris(dioxa-3,6 octyl)amine.

Dans un erlen de 100 $cm^3$ agité par un barreau magnétique sous atmosphère d'azote, on charge 7,9 g (0,05 mole) de m-chloronitrobenzène, 5,8 g (0,05 mole) de phénate de sodium, 0,5 g (0,005 mole) de chlorure cuivreux, 1,85 g (0,005 mole) de tris(dioxa-3,6 octyl)amine dans 50 $cm^3$ d'anisole. On chauffe à reflux pendant 6 h. Le taux de conversion atteint alors 72 %.

Essai comparatif

Le taux de transformation n'atteint que 12 % si on opère sans ajouter la tris(dioxa-3,6 octyl)amine.

Exemple 10

Préparation du métaphénoxybenzoate de méthyle.

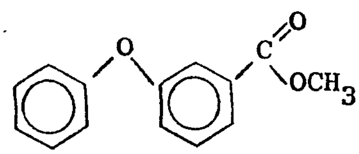

à partir de phénolate de sodium

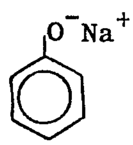

et de métachlorobenzoate de méthyle

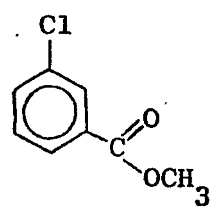

en présence de chlorure cuivreux Cu Cl et de tris(dioxa-3,6 octyl)amine.

Dans un ballon de 1 l équipé comme dans l'exemple 1, on charge 17,1 g (0,098 mole) de métachlorobenzoate de méthyle, 11,6 g (0,1 mole) de phénate de sodium, 1 g (0,001 mole) de chlorure cuivreux et 3,7 g (0,001 mole) de tris(dioxa-3,6 octyl)amine dans 300 g d'anisole. Après 6 h au reflux, le taux de transformation est de 75 %.

Essai comparatif

Le même essai conduit, sans tris(dioxa-3,6 octyl)amine ne permet qu'un taux de transformation de 1,5 %.

Exemple 11

Préparation de méthyl-2'phénoxy-1 trifluorométhyl-4 benzène

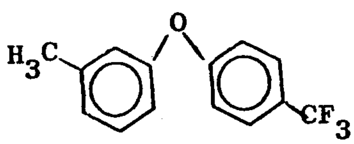

à partir de p-chlorotrifluorométhyl-benzène

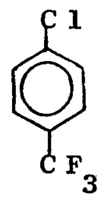

et de métacrésolate de sodium

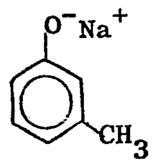

en présence de chlorure cuivreux Cu Cl et de tris(dioxa-3,6 heptyl)amine.

Dans un ballon de 1 l équipé comme dans l'exemple 1, on charge 65 g (0,5 mole) de m-crésolate de sodium, 90,25 g (0,5 mole) de p-chlorotrifluorométhyl-benzène, 5 g (0,005 mole) de chlorure cuivreux, 18 g (0,056 mole) de tris(dioxa-3,6 heptyl)amine et 300 g d'anisole. Après 6 h de reflux à 150 °C, le taux de transformation atteint 75 %.

Essai comparatif

Sans tris(dioxa-3,6 heptyl)amine, le taux de transformation n'est, après 6 h, que de 3,4 %.

Exemple 12

Préparation de métaphénoxytoluène

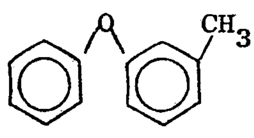

à partir de métacrésolate de sodium et de potassium

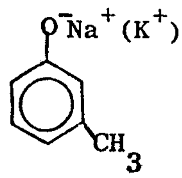

et de chlorobenzène

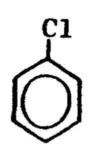

en présence de chlorure cuivreux et de tris(dioxa-3,6 octyl)amine.

Dans un appareil en acier inox de 100 l équipé d'une colonne à distiller, on charge 14,3 kg (132,4 moles) de m-crésol, 75 kg (667 moles) de chlorobenzène, 9,24 kg de soude 36° Bé, 4,48 kg de potasse 50 %. On opère la salification en distillant l'eau par azéotropie. Dès que la température de la masse atteint 132 °C, on charge 1,6 kg (16 moles) de chlorure cuivreux, 2 kg (5,5 moles) de tris(dioxa-3,6 octyl)amine et on fait passer un courant d'hydrogène. Après 5 h de reflux à 135 °C, le taux de conversion atteint 88 % et le rendement 93 %.

Après refroidissement à 80 °C, on extrait les sels formés à l'eau acide puis on soumet la masse organique à une distillation pour éliminer l'excès de chlorobenzène, le crésol non réagi, puis le m-phénoxytoluène. On obtient ainsi 21,6 kg de m-phénoxytoluène, soit un rendement de 89 %.

Exemple 13

Dans un erlenmeyer de 100 $cm^3$, agité par un barreau magnétique sous atmosphère d'azote, on charge 2,88 g du sel de sodium formé à partir du méta-hydroxy benzaldéhyde soit 0,02 mole, 60 g de bromobenzène, 0,2 g de Cu Cl (0,002 mole) et 0,15 g de tris(dioxa-3,6 octyl)amine. Le mélange est chauffé à 150 °C pendant 8 h. Le rendement de la réaction en méta-phénoxy-benzaldéhyde est de 75 %.

Essai comparatif

Le taux de transformation en méta-phénoxy-benzaldéhyde n'atteint que 1 % si on opère sans ajouter la tris(dioxa-3,6 octyl)amine.

Exemple 14

Préparation de la tris(dioxa-3,6 octyl)amine.

a) Dans un ballon tricol de 1 l, muni d'un agitateur mécanique, d'un thermomètre, d'un réfrigérant, on introduit 450 g d'éthoxy-2 éthanol (5 moles). On ajoute 23 g de sodium (1 mole), en 3 h en maintenant la température de mélange à 40 °C.

b) Au mélange précédent, on ajoute 51,6 g de chlorhydrate de tris(chloro-2 éthyl)amine (soit 0,215 mole). On chauffe alors le mélange à reflux pendant 12 h puis on distille le solvant sous pression réduite. Le ethoxy-2 ethanolate de sodium en excès est neutralisé par addition de 12 $cm^3$ d'H Cl aqueux (10 N). Le chlorure de sodium est filtré et la solution est distillée. La tris(dioxa-3,6 octyl)amine distille entre 200 °C et 210 °C sous 1 mmHg. Le rendement est de 68 %.

Exemple 15

Préparation de la tris(dioxa-3,6 heptyl)amine.

a) Dans un ballon tricol de 1 l, muni d'un agitateur mécanique, d'un thermomètre, d'un réfrigérant, on introduit 380 g de méthoxy-2 éthanol (5 moles). On ajoute 23 g de sodium (1 mole) en 3 h en maintenant la température de mélange à 40 °C.

b) au mélange précédent, on ajoute 51,6 g de chlorhydrate de tris(chloro-2 éthyl)amine (soit 0,215 mole). On chauffe alors le mélange à reflux (125 °C) pendant 12 h, puis on distille le solvant sous pression réduite. Le méthoxy-2 éthanolate de sodium en excès est neutralisé par addition de 11,6 $cm^3$ d'HCl aqueux (10 N). Le chlorure de sodium est filtré et la solution est distillée.

Exemple 16

Préparation de la tris(trioxa-3,6,9 decyl)amine.

Dans un ballon tricol de 1 l équipé d'un agitateur mécanique, d'un condenseur et d'un thermomètre, on introduit 600 g d'éther monométhylique du diéthylène glycol (dioxa-3,6 heptanol-1) soit 5 moles puis 23 g de sodium (1 mole) par petites fractions afin de former le dioxa-3,6 heptanolate de sodium.

Lorsque le sodium est totalement transformé, on ajoute alors 51,8 g de chlorhydrate de la tris(chloro-2 éthyl)amine (soit 0,215 mole). Le mélange est chauffé à 130 °C pendant 8 h sous agitation puis refroidi et l'excès d'alcoolate de sodium neutralisé par une solution aqueuse d'acide chlorhydrique à 10 %. Le dioxa-3,6 heptanol-1 est éliminé par distillation à 130 °C sous 20 mmHg. Le mélange obtenu est filtré afin d'éliminer le chlorure de sodium puis le produit est distillé. On obtient ainsi 83 g de tris(trioxa-3,6,9 décyl)amine qui distille à 189 °C sous 0,1 mmHg.

## Revendications

1. Procédé de préparation d'éther diarylique par réaction d'un halogénobenzène non activé et d'un phénolate alcalin en présence d'un composé du cuivre caractérisé en ce que la réaction a lieu en présence d'au moins un agent séquestrant de formule :

$$N—[CHR_1—CHR_2—O—(CHR_3—CHR_4—O)_n—R_5]_3 \qquad (I)$$

dans laquelle n est un nombre entier supérieur ou égal à 0 et inférieur ou égal à 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $—C_mH_{2m}—\emptyset$ ou $C_mH_{2m+1}—\emptyset—$, où m est compris entre 1 et 12.

2. Procédé selon la revendication 1, caractérisé en ce que dans la formule (I) $R_1$, $R_2$, $R_3$ et $R_4$ représentent un atome d'hydrogène ou un radical méthyle.

3. Procédé selon la revendication 1, caractérisé en ce que dans la formule (I) n est un nombre entier supérieur ou égal à 0 et inférieur ou égal à 6.

4. Procédé selon la revendication 1, caractérisé en ce que dans la formule (I) $R_5$ représente un radical alkyle ayant de 1 à 4 atomes de carbone.

5. Procédé selon les revendications 1-4, caractérisé en ce que dans la formule (I) $R_1$, $R_2$, $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

6. Procédé selon la revendication 5, caractérisé en ce que l'agent séquestrant de formule (I) est la tris(dioxa-3,6 heptyl)amine de formule :

$$N{-}(CH_2{-}CH_2{-}O{-}CH_2{-}CH_2{-}O{-}CH_3)_3$$

7. Procédé selon la revendication 5, caractérisé en ce que l'agent séquestrant de formule (I) est la tris(trioxa-3,6,9 décyl)amine de formule :

$$N{-}(CH_2{-}CH_2{-}O{-}CH_2{-}CH_2{-}O{-}CH_2{-}CH_2{-}O{-}CH_3)_3$$

8. Procédé selon la revendication 5, caractérisé en ce que l'agent séquestrant de formule (I) est la tris(dioxa-3,6 octyl)amine de formule :

$$N{-}(CH_2{-}CH_2{-}O{-}CH_2{-}CH_2{-}O{-}C_2H_5)_3$$

9. Procédé selon l'une quelconque des revendications précédentes, caractérisé en ce qu'on opère en présence d'un solvant choisi parmi le groupe comprenant l'oxyde de diphényle, l'anisole, le toluène, les xylènes, le polyéther de glycol, le benzène.

10. Procédé selon l'une quelconque des revendications 1 à 8, caractérisé en ce qu'on utilise l'agent séquestrant de formule I en quantité telle que le rapport molaire du composé du cuivre à l'agent séquestrant de formule I est compris entre 0,05 et 10.

11. Procédé selon la revendication 10, caractérisé en ce que le rapport molaire est compris entre 0,1 et 5.

12. Procédé selon l'une quelconque des revendications précédentes, caractérisé en ce qu'on opère à une température comprise entre 50 °C et 200 °C.

13. Procédé selon la revendication 12, caractérisé en ce que la température est comprise entre 100 et 180 °C.

14. Procédé de préparation selon l'une ou plusieurs des revendications précédentes du métaphénoxytoluène par réaction de métacrésolate de sodium et de chlorobenzène en présence de chlorure cuivreux caractérisé en ce qu'on opère en présence d'un agent séquestrant choisi parmi le groupe comprenant la tris(dioxa-3,6 octyl)amine et la tris(dioxa-3,6 heptyl)amine.

15. Procédé de préparation selon l'une ou plusieurs des revendications précédentes du métaphénoxytoluène par réaction de phénolate de sodium et de chloro-3 toluène en présence de chlorure cuivreux caractérisé en ce qu'on opère en présence de tris(dioxa-3,6 octyl)amine.

16. Procédé de préparation selon l'une ou plusieurs des revendications précédentes du métaphénoxybenzonitrile par réaction de chloro-3 benzonitrile et de phénolate de sodium en présence de chlorure cuivreux caractérisé en ce qu'on opère en présence de tris(dioxa-3,6 octyl)amine et dans un solvant choisi parmi le groupe comprenant l'anisole et l'oxyde de diphényle.

17. Procédé de préparation selon l'une ou plusieurs des revendications précédentes du métaphénoxybenzoate de méthyle par réaction de phénolate de sodium et de métachlorobenzoate de méthyle en présence de chlorure cuivreux caractérisé en ce qu'on opère en présence de tris(dioxa-3,6 octyl)amine dans l'anisole.

18. Procédé de préparation selon l'une ou plusieurs des revendications précédentes du métaphénoxybenzaldehyde par réaction du sel de sodium du métahydroxybenzaldehyde et de bromobenzène en présence de chlorure cuivreux caractérisé en ce qu'on opère en présence de tris(dioxa-3,6 octyl)amine.

**Claims**

1. Process for the preparation of a diaryl ether by reacting an unactivated halogenobenzene with an alkali metal phenolate, in the presence of a copper compound, characterised in that the reaction takes place in the presence of at least one sequestering agent of the formula :

$$N{-}[CHR_1{-}CHR_2{-}O{-}(CHR_3{-}CHR_4{-}O)_n{-}R_5]_3 \qquad (I)$$

in which n is an integer which is greater than or equal to 0 and less than or equal to 10 $(0 \leq n \leq 10)$, $R_1$, $R_2$, $R_3$ and $R_4$, which are identical or different, represent a hydrogen atom or an alkyl radical having from 1 to 4 carbon atoms and $R_5$ represents an alkyl or cycloalkyl radical having from 1 to 12 carbon atoms, a phenyl radical or a radical $-C_mH_{2m}-Ø$ or $C_mH_{2m+1}-Ø-$, in which m is between 1 and 12.

2. Process according to Claim 1, characterised in that, in the formula (I), $R_1$, $R_2$, $R_3$ and $R_4$ represent a hydrogen atom or a methyl radical.

3. Process according to Claim 1, characterised in that, in the formula (I), n is an integer which is greater than or equal to 0 and less than or equal to 3.

4. Process to Claim 1, characterised in that, in the formula (I), $R_5$ represents an alkyl radical having from 1 to 4 carbon atoms.

5. Process according to Claims 1-4, characterised in that, in the formula (I), $R_1$, $R_2$, $R_3$ and $R_4$, which are identical or different, represent a hydrogen atom or a methyl radical, n is an integer which is greater than or equal to 0 and less than or equal to 6 and $R_5$ represents an alkyl radical having from 1 to 4 carbon atoms.

6. Process according to Claim 5, characterised in that the sequestering agent of the formula (I) is tris(3,6-dioxaheptyl)-amine of the formula :

$$N—(CH_2—CH_2—O—CH_2—CH_2—O—CH_3)_3$$

7. Process according to Claim 5, characterised in that the sequestering agent of the formula (I) is tris-(3,6,9-trioxadecyl)-amine of the formula :

$$N—(CH_2—CH_2—O—CH_2—CH_2—O—CH_2—CH_2—O—CH_3)_3$$

8. Process according to Claim 5, characterised in that the sequestering agent of the formula (I) is tris(3,6-dioxaoctyl)-amine of the formula :

$$N—(CH_2—CH_2—O—CH_2—CH_2—O—C_2H_5)_3$$

9. Process according to any one of the preceding claims, characterised in that the reaction is carried out in the presence of a solvent chosen from amongst the group comprising diphenyl ether, anisole, toluene, xylenes, glycol polyether and benzene.

10. Process according to any one of Claims 1 to 8, characterised in that the amount of the sequestering agent of the formula I used is such that the molar ratio of the copper compound to the sequestering agent of the formula I is between 0.05 and 10.

11. Process according to Claim 10, characterised in that the molar ratio is between 0.1 and 5.

12. Process according to any one of the preceding claims, characterised in that the reaction is carried out at a temperature between 50 °C and 200 °C.

13. Process according to Claim 12, characterised in that the temperature is between 100 and 180 °C.

14. Process, according to one or more of the preceding claims, for the preparation of meta-phenoxytoluene by reacting sodium meta-cresolate with chlorobenzene, in the presence of cuprous chloride, characterised in that the reaction is carried out in the presence of a sequestering agent chosen from amongst the group comprising tris-(3,6-dioxaoctyl)-amine and tris-(3,6-dioxaheptyl)-amine.

15. Process, according to one or more of the preceding claims, for the preparation of meta-phenoxytoluene by reacting sodium phenolate with 3-chlorotoluene, in the presence of cuprous chloride, characterised in that the reaction is carried out in the presence of tris-(3,6-dioxaoctyl)-amine.

16. Process, according to one or more of the preceding claims, for the preparation of meta-phenoxybenzonitrile by reacting 3-chlorobenzonitrile with sodium phenolate, in the presence of cuprous chloride, characterised in that the reaction is carried out in the presence of tris-(3,6-dioxaoctyl)-amine and in a solvent chosen from amongst the group comprising anisole and diphenyl ether.

17. Process, according to one or more of the preceding claims, for the preparation of methyl meta-phenoxybenzoate by reacting sodium phenolate with methyl meta-chlorobenzoate, in the presence of cuprous chloride, characterised in that the reaction is carried out in the presence of tris-(3,6-dioxaoctyl)-amine in anisole.

18. Process, according to one or more of the preceding claims, for the preparation of meta-phenoxybenzaldehyde by reacting the sodium salt of meta-hydroxybenzaldehyde with bromobenzene, in the presence of cuprous chloride, characterised in that the reaction is carried out in the presence of tris-(3,6-dioxaoctyl)-amine.

**Ansprüche**

1. Verfahren zur Herstellung von Diaryläther durch Reaktion eines nicht aktiven Halogenbenzols und eines alkalischen Phenolates in Gegenwart einer Kupferverbindung, dadurch gekennzeichnet, daß die Reaktion in Gegenwart mindestens eines Sequestrierungsmittels der Formel :

$$N—[CHR_1—CHR_2—O—(CHR_3—CHR_4—O)_n—R_5]_3 \qquad (I)$$

durchgeführt wird, in der n eine ganze Zahl gleich oder grösser als 0 und gleich oder kleiner als 10

($0 \leq n \leq 10$) ist, $R_1$, $R_2$, $R_3$ und $R_4$ gleich oder unterschiedlich ein Wasserstoffatom oder einen Alkylrest mit 1 bis 4 Kohlenstoffatomen darstellen und $R_5$ einen Alkyl- oder Cycloalkylrest mit 1 bis 12 Kohlenstoffatomen, einen Phenylrest oder einen $—C_mH_{2m}—Ø$ oder $C_mH_{2m+1}—Ø$—Rest bedeuten, in dem m zwischen 1 und 12 liegt.

2. Verfahren nach Anspruch 1, dadurch gekennzeichnet, daß in der Formel (I) $R_1$, $R_2$, $R_3$ und $R_4$ ein Wasserstoffatom oder einen Methylrest bedeuten.

3. Verfahren nach Anspruch 1, dadurch gekennzeichnet, daß in der Formel (I) n eine ganze Zahl gleich oder größer als 0 gleich oder kleiner als 6 ist.

4. Verfahren nach Anspruch 1, dadurch gekennzeichnet, daß in der Formel (I) $R_5$ einen Alkylrest mit 1 bis 4 Kohlenstoffatomen bedeutet.

5. Verfahren nach den Ansprüchen 1 bis 4, dadurch gekennzeichnet, daß in der Formel (I) $R_1$, $R_2$, $R_3$ und $R_4$ gleich oder unterschiedlich ein Wasserstoffatom oder einen Methylrest bedeuten, n eine ganze Zahl gleich oder grösser als 0 gleich oder kleiner als 6 ist und $R_5$ ein Alkylrest mit 1 bis 4 Kohlenstoffatomen ist.

6. Verfahren nach Anspruch 5, dadurch gekennzeichnet, daß das Sequestrierungsmittel der Formel (I) das Tris-(dioxa-3,6-heptyl)amin der Formel :

$$N—(CH_2—CH_2—O—CH_2—CH_2—O—CH_3)_3$$

ist.

7. Verfahren nach Anspruch 5, dadurch gekennzeichnet, daß das Sequestrierungsmittel der Formel (I) das Tris(trioxa-3,6,9-decyl)amin der Formel :

$$N—(CH_2—CH_2—O—CH_2—CH_2—O—CH_2—CH_2—O—CH_3)_3$$

ist.

8. Verfahren nach Anspruch 5, dadurch gekennzeichnet, daß das Sequestrierungsmittel der Formel (I) das Tris(dioxa-3,6-octyl)amin der Formel :

$$N—(CH_2—CH_2—O—CH_2—CH_2—O—C_2H_5)_3$$

ist.

9. Verfahren nach irgendeinem der vorstehenden Ansprüche, dadurch gekennzeichnet, daß man in Gegenwart eines Lösungsmittels arbeitet, das aus der Diphenyloxid, Anisol, Toluol, Xylole, Polyglykoläther und Benzol umfassenden Gruppe ausgewählt ist.

10. Verfahren nach irgendeinem der Ansprüche 1 bis 8, dadurch gekennzeichnet, daß man das Sequestrierungsmittel der Formel (I) in einer solchen Menge benutzt, daß das Molverhältnis der Kupferverbindung zum Sequestrierungsmittel der Formel (I) zwischen 0,05 und 10 liegt.

11. Verfahren nach Anspruch 10, dadurch gekennzeichnet, daß das Molverhältnis zwischen 0,1 und 5 liegt.

12. Verfahren nach irgendeinem der vorstehenden Ansprüche, dadurch gekennzeichnet, daß man bei einer Temperatur zwischen 50 °C und 200 °C arbeitet.

13. Verfahren nach Anspruch 12, dadurch gekennzeichnet, daß die Temperatur zwischen 100 und 180 °C liegt.

14. Verfahren nach einem oder mehreren der vorstehenden Ansprüche zur Herstellung von Metaphenoxytoluol durch Umsetzung von Natriummetakresolat und Chlorbenzol in Gegenwart von Kupfer-(I)-chlorid, dadurch gekennzeichnet, daß man in Gegenwart eines Sequestrierungsmittels arbeitet, das aus der Tris(dioxa-3,6-octyl)amin und Tris(dioxa-3,6-heptyl)amin umfassenden Gruppe ausgewählt ist.

15. Verfahren nach einem oder mehreren der vorstehenden Ansprüche zur Herstellung von Metaphenoxytoluol durch Umsetzung von Natriumphenolat und 3-Chlortoluol in Gegenwart von Kupfer-(1)-chlorid, dadurch gekennzeichnet, daß man in Gegenwart von Tris(dioxa-3,6-octyl)amin arbeitet.

16. Verfahren nach einem oder mehreren der vorstehenden Ansprüche zur Herstellung von Metaphenoxybenzonitril durch Umsetzung von 3-Chlorbenzonitril und Natriumphenolat in Gegenwart von Kupfer-(1)-chlorid, dadurch gekennzeichnet, daß man in Gegenwart von Tris(dioxa-3,6-octyl)amin und in einem Lösungsmittel arbeitet, das aus der Anisol und Diphenyloxid umfassenden Gruppe ausgewählt ist.

17. Verfahren nach einem oder mehreren der vorstehenden Ansprüche zur Herstellung von Metaphenoxymethylbenzoat durch Umsetzung von Natriumphenolat und Metachlormethylbenzoat in Gegenwart von Kupfer-()-chlorid, dadurch gekennzeichnet, daß man in Gegenwart von Tris(dioxa-3,6-octyl)amin in Anisol arbeitet.

18. Verfahren nach einem oder mehreren der vorstehenden Ansprüche zur Herstellung von Metaphenoxybenzaldehyd durch Umsetzung des Natriumsalzes von Metahydroxybenzaldehyd und Brombenzol in Gegenwart von Kupfer-(1)-chlorid, dadurch gekennzeichnet, daß man in Gegenwart von Tris(dioxa-3,6-octyl)amin arbeitet.